# EUROPEAN PATENT SPECIFICATION

(11) **EP 3 632 411 B1**
(45) Date of publication and mention of the grant of the patent: **20.01.2021**
(21) Application number: 19201110.4
(22) Date of filing: 02.10.2019
(51) Int. Cl.: A61K 9/00, A61K 31/17, A61K 9/08, A61K 9/19, A61K 47/02, A61K 47/10, A61K 47/26, A61P 35/00

(54) **IMPROVED KIT FOR PREPARING INJECTABLE CARMUSTINE SOLUTIONS**
VERBESSERTER KIT ZUR HERSTELLUNG VON INJIZIERBAREN CARMUSTINLÖSUNGEN
KIT AMÉLIORÉ DE PRÉPARATION DE SOLUTIONS INJECTABLES DE CARMUSTINE

(30) Priority: 04.10.2018 IN 201821037526
(43) Date of publication of application: 08.04.2020
(73) Proprietor: Emcure Pharmaceuticals Limited, Pune 411026 (IN)
(72) Inventor: Gondaliya, Deepak Pragjibhai, 411026 Pune (IN); Patel, Hiren Pravinbhai, 411026 Pune (IN); Patel, Haresh Ishwarbhai, 411026 Pune (IN); Gurjar, Mukund Keshav, 411026 Pune (IN)
(74) Representative: Engelhard, Markus

(56) References cited:
- CN-A- 100 998 558
- CN-B- 102 370 983

## Description

### IMPROVED KIT FOR PREPARING INJECTABLE CARMUSTINE SOLUTIONS

The present application claims the benefit of Indian Patent Application No. 201821037526, October 4, 2018.

### FIELD OF THE INVENTION

The present invention relates to an improved kit for preparing injectable carmustine solutions and methods of preparing such solutions.

### BACKGROUND OF THE INVENTION

Carmustine (bischloroethyl nitrosurea also known as BCNU) is a nitrosurea drug for the treatment of brain cancers owing to its ability to cross the blood-brain barrier and excellent activity against brain tumours.

Carmustine chemically known as 1,3-bis(2-chloroethyl)-1-nitrosourea (shown below) alkylates DNA, RNA and interferes with its synthesis and functions. It also binds and modifies (carbamoylates) glutathione reductase, which consequently leads to cell death.

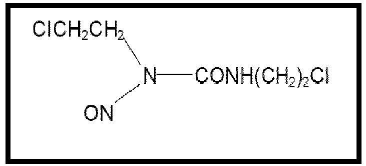

Carmustine is poorly soluble in water and is unstable in many formulations. For instance, carmustine gets readily hydrolyzed in water at pH >6. The solubility and stability issues of carmustine have been discussed previously. *See,* for example, Levin et al., Selective Cancer Therapeutics, 1989, 5(1), 33-35.

Carmustine is commercially available as a lyophilized 100 mg powder for injection under the trade name BiCNU® in single dose vials. *See* the March 2017 prescribing information for BiCNU®, which is hereby incorporated by reference. Ethanol (dehydrated alcohol) (3 mL) is co-packaged with the drug product as a sterile diluent for reconstitution. To prepare the drug for administration, three preparation steps need to be performed. First, the lyophilized carmustine is dissolved with the co-packed sterile dehydrated alcohol (3 mL) diluent. Second, the solution is further diluted with 27 mL of sterile water to form the reconstituted solution. Third, the reconstituted solution is further diluted with 5% Dextrose Injection, USP or Sodium Chloride Injection, USP (0.9% sodium chloride). This complicated preparation of carmustine solutions is time-consuming and can to lead to errors in preparation and dosing.

Furthermore, ethanol in injectable products may cause undesirable adverse events, including infusion toxicity and hypersensitivity reactions. The U.S. Food and Drug Administration (FDA) in June 2014 issued a warning that the cancer drug docetaxel may cause symptoms of alcohol intoxication after treatment. *See* https://www.fda.gov/drugs/drug-safety-and-availability/fda-drug-safety-communication-fda-warns-cancer-drug-docetaxel-may-cause-symptoms-alcohol. The warning was issued in response to several instances of patients being intoxicated after receiving docetaxel. In two cases doctors decided to use different formulations of the drug with lower alcohol content for subsequent treatments. Sanofi Aventis which markets docetaxel as TAXOTERE revised the package insert by adding alcohol content to the Warnings and Precautions section. Specifically, the package insert refers to the cases of intoxication and warns that the alcohol content in a dose of TAXOTERE is likely to affect the central nervous system and should be controlled for patients in whom alcohol intake should be avoided or minimized.

Due to the known toxicity of ethanol, Layton et al., J. Neurosurgery, 1984, 60(6), 1134-1137, attempted to reduce the amount of ethanol diluent used for reconstitution of carmustine from 3 mL to 2 mL or 0.75 mL (per 100 mg of carmustine). The amount of carmustine recovered *in vitro* after simulated clinical administration of the three solutions decreased from 84.9% to 38.3% as the diluent decreased. Clarity and homogeneity decreased as the ethanol content decreased. As an alternative, Layton attempted to dissolve 500 mg of carmustine in 150 mL of 5% dextrose in water. This required 30 minutes of vigorous shaking before there was no visible powder, rendering it impractical for clinical use. Patent document CN 102370983 discloses formulations comprising carmustine and propylene glycol.

Hence, there is an ongoing need for improved kits which simplify the preparation and improve the safety of injectable carmustine solutions.

### SUMMARY OF THE INVENTION

The present inventors surprisingly found that certain ethanol-free non-aqueous diluents, such as propylene glycol, can be used for reconstitution of lyophilized carmustine. These diluents were found to improve the solubility of carmustine. As a result, a simpler single-step process can be used to prepare injectable carmustine solutions according to the claims from lyophilized carmustine.

Furthermore, a preferred ethanol-free non-aqueous diluent, propylene glycol, was found to result in a reconstituted solution having improved stability compared to reconstitution of carmustine in ethanol.

One embodiment is a kit comprising a product vial containing lyophilized carmustine and a diluent vial containing ethanol-free non-aqueous diluent, propylene glycol. Preferably, the product vial contains only lyophilized carmustine. In one embodiment, the lyophilized carmustine does not contain a bulking agent, such as mannitol. The diluent vial only contains the ethanol-free non-aqueous diluent, propylene glycol.

The product vial may contain 50-200 mg of lyophilized carmustine, and the diluent vial may contain 2-6 mL of the ethanol-free non-aqueous diluent, propylene glycol. In a preferred embodiment, the product vial contains 100 mg of lyophilized carmustine and the diluent vial contains 3 mL of ethanol-free non-aqueous diluent, propylene glycol (more preferably, sterile propylene glycol).

Another embodiment is a method of preparing an administrable solution of carmustine comprising (a) dissolving lyophilized carmustine in an ethanol-free, non-aqueous propylene glycol as diluent to form a reconstituted solution, and (b) diluting the reconstituted solution with an aqueous 0.9% sodium chloride solution (preferably Sodium Chloride Injection, USP) or an aqueous 5% dextrose solution (preferably 5% Dextrose Injection, USP) to obtain the administrable solution. This method includes a single reconstitution step unlike the current procedure required for BiCNU® which includes two steps to reconstitute the carmustine (i.e., dissolution in 3 mL of ethanol followed by further dissolution in 27 mL of water). The reconstituted solution of the present invention has superior stability compared to reconstitution with 3 mL of ethanol.

In one embodiment, the administrable solution is prepared by (a) dissolving 50-200 mg (e.g., 100 mg) of lyophilized carmustine in 2-6 mL (e.g., 3 mL) of propylene glycol (e.g., sterile propylene glycol or propylene glycol USP) to form a reconstituted solution, and (b) diluting the reconstituted solution with an aqueous 0.9% sodium chloride solution or an aqueous 5% dextrose solution to obtain the administrable solution. In a preferred embodiment, the reconstituted solution is stable (≥ 90% of carmustine remaining) after storage at 2-8° C for up to 720 hours or at 25 °C ± 2 °C for 24 or 48 hours. Step (b), for example, may include diluting the reconstituted solution up to 500 mL with an aqueous 0.9% sodium chloride solution (preferably Sodium Chloride Injection, USP) or an aqueous 5% dextrose solution (preferably 5% Dextrose Injection, USP). Step (b) is preferably performed within 480 hours of the reconstituted solution being prepared, where the reconstituted solution is stored at 2-8° C. After storage at 2-8° C and prior to performing step (b), the reconstituted solution is preferably examined for crystal formation and if crystals are observed, they may be re-dissolved by warming the re-constituted solution to room temperature optionally with agitation. When the reconstituted solution is stored at room temperature, step (b) is preferably performed within 48 hours of the reconstituted solution being prepared. For instance, step (b) may be performed more than 24 hours but less than 480 or 48 hours after the reconstituted solution is prepared. The lyophilized carmustine and the ethanol-free, non-aqueous diluent may be from a kit as described herein. In one preferred embodiment, the administrable solution has a pH in the range of 6 to 7 and an osmolality in the range of 330-390 mOsmol/L.

Yet another embodiment is a method for administering carmustine to a patient in need thereof by administering by intravenous infusion an administrable solution of carmustine, where the administrable solution is prepared from a kit comprising a product vial containing 100 mg of lyophilized carmustine and a diluent vial containing 3 mL of sterile propylene glycol, and the kit is stored at 2-8° C. The administrable solution is prepared by:
(a) allowing the diluent vial to attain room temperature,
(b) aseptically removing the 3 mL of propylene glycol from the diluent vial (preferably with a below 22-gauge needle), injecting it into the product vial containing 100 mg of lyophilized carmustine, and shaking the product vial to dissolve the lyophilized carmustine to form a reconstituted solution,
(c) optionally, storing the reconstituted solution and prior to performing step (d), the stored reconstituted solution is examined for crystal formation and if crystals are observed, they are re-dissolved by warming the reconstituted solution to room temperature with agitation, and
(d) diluting the reconstituted solution up to 500 mL with an aqueous 0.9% sodium chloride solution or an aqueous 5% dextrose solution to obtain the administrable solution.

Also disclosed is a method of administering carmustine comprising intravenously administering an administrable carmustine solution as described herein to a patient in need thereof. The administrable carmustine solution may be prepared as described herein. The scope of the invention is defined by the claims. Any references in the description to methods of treatment refer to the compounds, pharmaceutical compositions and medicaments of the present invention for use in a method for treatment of the human (or animal) body by therapy (or for diagnosis).

Also disclosed is a method of treating cancer in a patient in need thereof by intravenously administering an administrable carmustine solution as described herein to the patient. The administrable carmustine solution may be prepared as described herein. The patient may be suffering from brain tumors glioblastoma, brainstem glioma, medulloblastoma, astrocytoma, ependymoma, metastatic brain tumors, multiple myeloma, relapsed or refractory Hodgkin's lymphoma, or relapsed or refractory Non-Hodgkin's lymphomas.
In particular, the invention may be illustrated by the following embodiments:
In one aspect the present invention relates to a method for preparing an administrable solution of carmustine for administration by intravenous infusion, said method comprising:
(a) dissolving 50-200 mg of lyophilized carmustine in 2-6 mL of sterile propylene glycolto form a reconstituted solution, and
(b) diluting the reconstituted solution with an aqueous 0.9% sodium chloride solution or an aqueous 5% dextrose solution to obtain the administrable solution.

In one embodiment, step (a) comprises dissolving 100 mg of lyophilized carmustine in 3 mL of sterile propylene glycol to form a reconstituted solution.

In one embodiment, prior to dissolving the lyophilized carmustine in the propylene glycol, (i) the lyophilized carmustine and propylene glycol are stored in separate vials at 2-8° C and either (ii) the propylene glycol is allowed to attain room temperature just prior to dissolving the lyophilized carmustine in the propylene glycol, or (ii') the vials are allowed to attain room temperature just prior to dissolving the lyophilized carmustine in the propylene glycol.

In one embodiment, wherein the propylene glycol is aseptically removed from its vial with a sterile syringe having a needle below 22 gauge and injected into the vial containing the lyophilized carmustine.

In one embodiment, the reconstituted solution contains at least 90% of the initial carmustine after storage at 2-8° C for up to 480 hours.

In one embodiment, the reconstituted solution is stored at 2-8° C for up to 480 hours prior to step (b), and after storage at 2-8° C and prior to performing step (b), the reconstituted solution is examined for crystal formation and if crystals are observed, they are re-dissolved by warming the re-constituted solution to room temperature with agitation.

In one embodiment, step (b) comprises diluting the reconstituted solution up to 500 mL with an aqueous 0.9% sodium chloride solution or 500 mL of an aqueous 5% dextrose solution to obtain the administrable solution.

In one embodiment, step (b) is performed within 48 hours of the reconstituted solution being prepared, and/or wherein step (b) is performed more than 24 hours but less than 48 hours after the reconstituted solution is prepared.

In one embodiment, the concentration of carmustine in the administrable solution is 0.2 mg/mL, and/or wherein administrable solution has a pH in the range of 6 to 7 and an osmolality in the range of 330-390 mOsmol/L.

In a further aspect, the present invention relates to a method for preparing an administrable solution of carmustine for administration by intravenous infusion, wherein the administrable solution is prepared from a kit comprising a product vial containing 100 mg of lyophilized carmustine and a diluent vial containing 3 mL of sterile propylene glycol, the kit being stored at 2-8° C, and wherein the administrable solution is prepared by:
(a) allowing the diluent vial to attain room temperature,
(b) aseptically removing the 3 mL of propylene glycol from the diluent vial, injecting it into the product vial containing 100 mg of lyophilized carmustine, and shaking the product vial to dissolve the lyophilized carmustine to form a reconstituted solution, wherein, preferably, the lyophilized carmustine from the product vial is reconstituted with specified amount of propylene glycol from the diluent vial in a single-step reconstitution procedure,
(c) optionally, storing the reconstituted solution and prior to performing step (d), the stored reconstituted solution is examined for crystal formation and if crystals are observed, they are re-dissolved by warming the reconstituted solution to room temperature with agitation, and
(d) diluting the reconstituted solution up to 500 mL with an aqueous 0.9% sodium chloride solution or an aqueous 5% dextrose solution to obtain the administrable solution.

In one embodiment, the propylene glycol is removed from the diluent vial in step (b) with a below 22-gauge needle.

In a further aspect the present invention relates to a kit for preparing an injectable carmustine solution, the kit comprising a product vial containing lyophilized carmustine and a diluent vial containing sterile propylene glycol, wherein, upon dissolving the lyophilized carmustine in the propylene glycol, the resulting solution contains at least 90% of the initial carmustine after storage at 2-8° C for up to 720 hours or at 25 °C ± 2 °C for up to 48 hours.

In one embodiment of the kit, the concentration of lyophilized carmustine in the product vial is from about 2 mg/vial to about 500 mg/vial, wherein, preferably, the product vial contains 50-200 mg of lyophilized carmustine and the diluent vial contains 2-4 mL of sterile propylene glycol.

In one embodiment, the amount of sterile propylene glycol in the diluent vial is between 1 ml and 20 ml, preferably 2-4 mL.

In yet a further aspect the present invention relates to an administrable solution of carmustine for administration by intravenous infusion, preferably produced by a method as defined further above, said administrable solution comprising carmustine and propylene glycol, wherein the carmustine is reconstituted in propylene glycol, and further comprising water and either sodium chloride or dextrose.

In one embodiment, the concentration of carmustine in the administrable solution is 0.2 mg/mL, and/or wherein the administrable solution has a pH in the range of 6 to 7 and an osmolality in the range of 330-390 mOsmol/L.

In yet another aspect, the present invention relates to the administrable solution of carmustine as defined further above, for use in a method of treating cancer in a patient, said method comprising intravenously administering said administrable solution to said patient, wherein, preferably, said cancer is selected from brain tumor glioblastoma, brainstem glioma, medulloblastoma, astrocytoma, ependymoma, metastatic brain tumors, multiple myeloma, relapsed or refractory Hodgkin's lymphoma, and relapsed or refractory Non-Hodgkin's lymphoma.

### DETAILED DESCRIPTION OF THE INVENTION

The terms "ethanol" and "dehydrated alcohol" are used synonymously throughout the specification.

The U.S. Pharmacopeia, USP 42 - NF 37 (2019) discloses entries for Sodium Chloride Injection, USP, 5% Dextrose Injection, USP.

### Kit

One embodiment is a kit comprising a product vial containing lyophilized carmustine and a diluent vial containing ethanol-free non-aqueous diluent. Preferably, the product vial contains only lyophilized carmustine. In one embodiment, the lyophilized carmustine does not contain a bulking agent. The amount of lyophilized carmustine in product vial may vary from about 2 mg/vial to about 500 mg/vial, preferably 50 mg/vial, 100 mg/vial and 300 mg/vial. The lyophilized carmustine, which typically is in the form of a powder, may be prepared by methods known in the art, such as those described in U.S. Patent Publication No. 2016/0136116.

The diluent vial only contains the ethanol-free non-aqueous diluent, propylene glycol. Generally, other ethanol free non-aqueous diluents include, but are not limited to, aliphatic amides (such as N,N-dimethylacetamide and N-hydroxy-2-ethyl-lactamide), glycols and polyalcohols (such as propylene glycol and glycerine), esters of polyalcohols (such as diacetine (glyceryl diacetate), triacetine (glyceryl triacetate)), polyglycols and polyethers (such as propylene glycol methyl ethers), transcutol, dioxolanes (such as isopropylidene glycerine), N-methylpyrrolidone, or any combination of any of the foregoing. According to one preferred embodiment, the ethanol-free non-aqueous diluent is propylene glycol, N,N-dimethylacetamide, transcutol, or methylpyrrolidone. The ethanol-free non-aqueous diluent is preferably sterile. According to the claims, the ethanol-free non-aqueous diluent is propylene glycol, such as sterile propylene glycol or propylene glycol USP.

The amount of ethanol-free non-aqueous diluent in the diluent vial may vary from between 1 ml and 20 ml. Preferably the amount of non-aqueous diluent is 1 ml, 2 ml, 3 ml, 4 ml, 5 ml, 6 ml, 7 ml, 8 ml, 9 ml, 10 ml, 11ml, 12 ml, 13 ml, 14 ml or 15 ml, and more preferably is 3 ml.

In one embodiment, the product vial contains 50-200 mg of lyophilized carmustine, and the diluent vial contains 2-6 mL of the ethanol-free non-aqueous diluent, propylene glycol.

In a preferred embodiment, the product vial contains 100 mg of lyophilized carmustine and the diluent vial contains 3 mL of ethanol-free non-aqueous diluent, propylene glycol (more preferably, sterile propylene glycol or propylene glycol USP).

The vials are preferably made of glass or polypropylene (such as polypropylene which is polyvinyl chloride (PVC) free and di-2-ethylhexyl phthalate (DEHP) free). The vials are preferably not made of (and do not contain) polyvinyl chloride.

In a preferred embodiment, the product vial is stored at 2-8° C. In another preferred embodiment, the product vial and diluent vial are stored at 2-8° C.

### Preparation

In another embodiment, the present invention provides a single-step reconstitution procedure for carmustine injection wherein the lyophilized carmustine, such as from the product vial, is reconstituted with a specified amount of the ethanol-free non-aqueous diluent, such as from the diluent vial.

This reconstitution procedure of the present invention is advantageous over the current procedure used for BiCNU® as it requires a single-step dilution with an ethanol-free non-aqueous diluent. In other words, the additional step of dilution with 27 mL of water for injection as described in the current package insert for BiCNU® is eliminated.

Prior to reconstitution, the diluent vial may be allowed to attain room temperature, for example, by removal from a refrigerator (where it is stored at 2-8° C). In one embodiment, both the product vial and diluent vial are removed from a refrigerator (where they are stored at 2-8° C) and allowed to attain room temperature. In one embodiment, the propylene glycol is removed from the diluent vial using an appropriate needle (e.g., a 22-gauge needle or a needle below 22 gauge). In one preferred embodiment, the needle is below 22 gauge. In one embodiment, the propylene glycol is aseptically removed from the diluent vial with a sterile syringe and injected into the product vial containing carmustine. The product vial may be gently shaken to dissolve the carmustine.

The typical two-step reconstitution procedure for the current BiCNU® product (as per its package insert) is as described below:
1) Aseptically removing 3 mL of ethanol diluent from the diluent vial using a sterile syringe and injecting it into the product vial containing the lyophilized carmustine, followed by gentle shaking to obtain a clear solution, and
2) Aseptically adding 27 mL of sterile water for injection into the solution of step (1), followed by gentle shaking to obtain a clear solution.

The single-step reconstitution procedure of the present invention, in contrast, can be as described below:
1) Aseptically removing an appropriate quantity (e.g. 3 mL) of ethanol-free non-aqueous diluent (e.g., propylene glycol) from the diluent vial using a sterile syringe and injecting it into the product vial containing the lyophilized carmustine, followed by gentle shaking to obtain a clear solution.

Preferably, the diluent propylene glycol is allowed to attain room temperature before it is aseptically removed from its vial and injected into the product vial. Preferably, the lyophilized carmustine dissolves in the propylene glycol within 3 minutes and more preferably within 2 minutes.

In one embodiment, the reconstituted carmustine solution has a concentration of about 33.3 mg/mL of carmustine.

Prior to administration, the reconstituted carmustine solution may be further admixed with 0.9% sodium chloride injection or 5% dextrose injection to form an administrable solution. For instance, in one embodiment, the reconstituted carmustine solution is further diluted up to 500 mL with 0.9% sodium chloride injection or 5% dextrose injection.

The reconstituted carmustine solution may be stored at room temperature or at 2-8° C prior to being admixed with the 0.9% sodium chloride injection or 5% dextrose injection. The admixing step is preferably performed within 480 hours of the reconstituted solution being prepared, where the reconstituted solution is stored at 2-8° C. After storage at 2-8° C and prior to being admixed, the reconstituted solution is preferably examined for crystal formation and if crystals are observed, they may be re-dissolved by warming the re-constituted solution to room temperature optionally with agitation. When the reconstituted solution is stored at room temperature, the admixing step is preferably performed within 48 hours of the reconstituted solution being prepared. For instance, the admixing step may be performed more than 24 hours but less than 480 or 48 hours after the reconstituted solution is prepared.

The reconstituted solution and/or administrable solution may be stored in a glass or polypropylene container (such as a polypropylene container which is polyvinyl chloride (PVC) free and di-2-ethylhexyl phthalate (DEHP) free). These solutions are preferably not stored in a polyvinyl chloride container.

The administrable solution may be a faint yellow colour with a pH in the range of 6 to 7 and osmolality in the range of 330-390 mOsmol/L. In a preferred embodiment, the administrable solution has a pH of 6.5 and osmolality of 350-380 mOsmol/L.

The administrable carmustine solution can have a concentration of about 0.2 mg/mL carmustine.

In another embodiment, the reconstituted carmustine solution has improved stability over the reconstituted carmustine solution of the reference product.

As used herein, a "stable" reconstituted carmustine solution means no aggregation was observed when stored at 2 to 8° C (long-term storage condition) and 25°C±2° C (accelerated storage condition) for an appropriate time and where the assay of carmustine is >90%.

The carmustine content after storage is determined by high performance liquid chromatography (HPLC method). HPLC was used for performing the assay studies described in the examples below.

Based on the results of Tables 3 and 4 below, it was concluded that the reconstituted carmustine solution of the present invention was stable for up to 720 hours (e.g., for up to 480 hours) when stored at 2°C - 8°C and for up to 48 hours when stored at 25°C±2°C. In contrast, the reconstituted carmustine solution of the reference product was stable only under refrigerated conditions (2°C-8°C) for up to 96 hours. Accordingly, propylene glycol was found to be superior as a diluent over the dehydrated alcohol diluent of the reference product.

The stability of the admixed carmustine solution was also performed separately at 2°C to 8°C (long-term storage condition) for an appropriate time, 25°C±2°C (accelerated storage condition) for appropriate time and 2°C to 8°C for appropriate time followed by 25°C±2°C for appropriate time.

Based on the results of Tables 5 and 6, it was concluded that the admixed carmustine solution of the present invention was stable for 48 hours (2-8°C) + 6 hours (25°C±2°C).

### Administration

The carmustine administrable solution may be administered to a patient (e.g., a human patient) by slow intravenous infusion over at least two hours. In one embodiment, the injected area is monitored during the administration. In another embodiment, the rate of administration of the intravenous infusion is no more than 1.66 mg/m²/min. The patient may suffer from cancer.

In one embodiment, the carmustine administrable solution may be administered to a patient to treat brain tumors glioblastoma, brainstem glioma, medulloblastoma, astrocytoma, ependymoma, metastatic brain tumors, multiple myeloma, relapsed or refractory Hodgkin's lymphoma, or relapsed or refractory Non-Hodgkin's lymphomas.

In one embodiment, the carmustine administrable solution is administered to a patient as a single agent or in a combination therapy (such as with other chemotherapeutic agents) to treat (i) brain tumors glioblastoma, brainstem glioma, medulloblastoma, astrocytoma, ependymoma, or metastatic brain tumors, (ii) multiple myeloma in combination with prednisone, (iii) relapsed or refractory Hodgkin's lymphoma in combination with other approved drugs (such as chemotherapeutic agents), or (iv) relapsed or refractory Non-Hodgkin's lymphomas in combination with other approved drugs (such as chemotherapeutic agents).

The carmustine administrable solution may be administered as a single agent in previously untreated patients at a dose of 150 to 200 mg/m² carmustine intravenously every 6 weeks. The carmustine administrable solution may be administered as a single dose or divided into daily injections such as 75 to 100 mg/m² on two successive days. The dose may be lowered when the carmustine administrable solution is used with other myelosuppressive drugs or in patients in whom bone marrow reserve is depleted. The carmustine administrable solution may be administered for the duration according to the established regimen. In one embodiment, the patient is premedicated before each dose with antiemetics.

The dosing (after the initial dose) may be adjusted according to the hematologic response of the patient to the preceding dose. In one embodiment, the patient is dosed as follows:

| Nadir After Prior Dose | | Percentage of Prior Dose to be Given |
|---|---|---|
| Leuko cytes/mm³ | Platelets/mm³ | |
| >4000 | >100,000 | 100% |
| 3000-3999 | 75,000-99,999 | 100% |
| 2000-2999 | 25,000-74,999 | 70% |
| <2000 | <25,000 | 50% |

The hematologic toxicity can be delayed and cumulative. In one embodiment, the patient's blood counts are monitored weekly. In another embodiment, a repeat course of the carmustine administrable solution is not administered until circulating blood elements have returned to acceptable levels (platelets above 100 Gi/L, leukocytes above 4 Gi/L and absolute neutrophil count above 1 Gi/L). In yet another embodiment, the interval between courses is 6 weeks.

In yet another embodiment, renal function is evaluated prior to administration and/or periodically during treatment. In one embodiment, carmustine treatment is discontinued if the creatinine clearance is less than 10 mL/min. In another embodiment, carmustine is not administered to patients with compromised renal function. In yet another embodiment, transaminases and bilirubin are monitored periodically during treatment.

The following examples further illustrate the invention but should not be construed as in any way limiting its scope. In particular, the processing conditions are merely exemplary and can be varied by one of ordinary skill in the art.

### EXAMPLES

In the examples and tables below, the following terms and abbreviations have the specified definitions.
"IA" refers to Impurity A.
"Impurity A" refers to 1,3-bis(2-chloroethyl)urea.
"IUUI" refers to an individual unspecified unidentified impurity.
"TI" refers to total impurities.

The content of carmustine and impurities was determined by high performance liquid chromatography (HPLC).

### Comparative Example 1 (Reference Product)

**Table 1: Composition of BICNU® (Reference product)**

| **Composition** | **Quantity** |
|---|---|
| **Product Vial** | |
| Lyophilized Carmustine | 100 mg |

| **Diluent Vial** | |
|---|---|
| Sterile Dehydrated Alcohol (DHA) | 3.0 ml |

The components in Table 1 above were used to prepare a reconstituted carmustine solution. The reconstitution procedure as described below was followed:
1) Aseptically removing 3 mL of ethanol diluent from the diluent vial using a sterile syringe and injecting it into the product vial containing the lyophilized carmustine, followed by gentle shaking to obtain a clear solution, and
2) Aseptically adding 27 mL of sterile water for injection into the solution of step (1), followed by gentle shaking to obtain a clear solution.

The stability of the reconstituted carmustine solution at 2-8° C for 720 hours and at 25 ± 2° C for 120 hours was evaluated. The results are provided in Tables 3 and 4.

### Test Example 1

**Table 2: Composition of carmustine for injection**

| **Composition** | **Quantity** |
|---|---|
| **Product Vial** | |
| Lyophilized Carmustine | 100 mg |

| **Diluent Vial** | |
|---|---|
| Sterile Propylene Glycol (PG) | 3.0 ml |

The product vial and the diluent vial were removed from a refrigerator and allowed to attain room temperature. The single-step reconstitution procedure was performed as follows. 3 mL of propylene glycol was aseptically removed from the diluent vial using a sterile syringe and injected into the product vial containing lyophilized carmustine. The product vial was gently shaken to form a clear solution. The stability of the reconstituted carmustine solution at 2-8° C for 720 hours and at 25 ± 2° C for 120 hours was evaluated. The results are provided in Tables 3 and 4.

**Table 3: Stability data of reconstituted carmustine solution with propylene glycol (PG) and dehydrated alcohol (DHA) stored at 2°C - 8°C**

| **Sampling intervals (Hrs.)** | **Assay (%)** | | **Related Substance (%)** | | | | | |
|---|---|---|---|---|---|---|---|---|
| | **PG** | **DHA** | **PG** | | | **DHA** | | |
| | | | **Impurity A** | **IUUI** | **Total Impurities** | **Impurity A** | **IUUI** | **Total Impurities** |
| 0 | 99.8 | 98.2 | 0.20 | BLQ | 0.20 | 0.20 | 0.018 | 0.22 |
| 12 | 100 | 96.8 | 0.18 | 0.023 | 0.21 | 0.20 | 0.02 | 0.24 |
| 24 | 101.4 | 96.4 | 0.18 | ND | 0.18 | 0.22 | 0.038 | 0.28 |
| 48 | 101.9 | 94.0 | 0.17 | BLQ | 0.17 | 0.18 | 0.090 | 0.30 |
| 96 | 100.7 | 90.1 | 0.19 | 0.004 | 0.20 | 0.17 | 0.176 | 0.38 |
| 144 | 100.9 | 85.6 | 0.27 | 0.005 | 0.28 | 0.23 | 0.204 | 0.46 |
| 192 | 99.7 | 80.2 | 0.34 | 0.006 | 0.34 | 0.25 | 0.239 | 0.52 |
| 240 | 98.2 | 78.3 | 0.34 | 0.016 | 0.40 | 0.23 | 0.284 | 0.56 |
| 480 | 99.1 | 63.2 | 0.82 | 0.008 | 0.83 | 0.41 | 0.302 | 0.78 |
| 720 | 96 | 51.1 | 1.54 | 0.011 | 1.57 | 0.61 | 0.285 | 0.97 |

**Table 4: Stability data of reconstituted carmustine solution with propylene glycol (PG) and dehydrated alcohol (DHA) stored at 25°C ± 2°C**

| **Sampling intervals (Hrs.)** | **Assay (%)** | | **Related Substance (%)** | | | | | |
|---|---|---|---|---|---|---|---|---|
| | **PG** | **DHA** | **PG** | | | **DHA** | | |
| | | | **Impurity A** | **IUUI** | **Total Impurities** | **Impurity A** | **IUUI** | **Total Impurities** |
| 0 | 99.8 | 98.2 | 0.20 | BLQ | 0.20 | 0.20 | 0.018 | 0.22 |
| 12 | 97.9 | 80.8 | 0.27 | 0.010 | 0.28 | 0.22 | 0.215 | 0.48 |
| 24 | 95.5 | 65.4 | 0.48 | 0.004 | 0.48 | 0.27 | 0.299 | 0.63 |
| 48 | 94.3 | 45.9 | 1.41 | 0.005 | 1.42 | 0.41 | 0.368 | 0.86 |
| 96 | 87.0 | 20.1 | 3.86 | 0.031 | 3.89 | 0.41 | 0.269 | 0.92 |
| 120 | 82.9 | 13.4 | 6.39 | 0.009 | 6.41 | 0.41 | 0.151 | 0.67 |

As shown by Tables 3 and 4, Test Example 1 was stable (≥ 90% carmustine remaining) for 720 hours when stored at 2°C-8°C and for up to 48 hours when stored at 25°C±2°C. In contrast, the Reference Product was stable (≥ 90% carmustine remaining) only under refrigerated conditions (2°C-8°C) for up to 96 hours.

The reconstituted carmustine solution of Test Example 1 was further admixed with 500 mL of 0.9% sodium chloride injection or 5% dextrose injection to form an admixed carmustine solution for clinical use. The stability of the admixed carmustine solution at (i) 2-8° C for 24 hours followed by 25 ± 2° C for 12 hours, (ii) 2-8° C for 48 hours followed by 25 ± 2° C for 12 hours, and (iii) 25 ± 2° C for 8 hours was evaluated. The results are provided in Tables 5 and 6.

**Table 5: Assay of admixed carmustine solution with 0.9% sodium chloride injection (NaCl) and 5% dextrose injection**

| **Sampling intervals & Storage condition** | **Assay (%)** | | | |
|---|---|---|---|---|
| | **PG** | | **DHA** | |
| | **NaCl** | **Dextrose** | **NaCl** | **Dextrose** |
| Stored up to 24 hours at 2°C-8°C and further up to 12 hours at 25°C ± 2°C | | | | |
| Initial (0 hour) | 102.9 | 100.7 | 103.7 | 100.6 |
| 12hrs. (2-8°C) | 100.7 | 99.1 | 101.4 | 99.1 |
| 24hrs. (2-8°C) | 99.0 | 98.9 | 99.8 | 98.7 |
| 24hrs. (2-8°C) + 6hrs. (25 °C ± 2°C) | 94.3 | 94.5 | 94.7 | 94.2 |
| 24hrs. (2-8°C) + 12hrs. (25°C ± 2°C) | 87.3 | 88.1 | 87.2 | 88.0 |

| Stored up to 48 hours at 2°C-8°C and further up to 12 hours at 25°C ± 2°C | | | | |
|---|---|---|---|---|
| Initial (0 hour) | 103.4 | 104.4 | 104.4 | 102.8 |
| 48 hrs. (2-8°C) | 97.2 | 97.8 | 97.5 | 96.7 |
| 48 hrs.(2-8°C) + 6 hrs. (25°C ± 2°C) | 92.9 | 93.3 | 92.7 | 92.1 |
| 48 hrs. (2-8°C) + 12 hrs. (25°C ± 2°C) | 84.9 | 88.0 | 84.4 | 84.9 |

| Stored up to 8 hours at 25°C ± 2°C | | | | |
|---|---|---|---|---|
| Initial (0 hour) | 103.4 | 103.9 | 104.0 | 101.8 |
| 4 hrs. (25°C ± 2°C) | 96.2 | 99.1 | 97.2 | 96.3 |
| 8 hrs. (25 ± 2°C) | 86.4 | 92.5 | 88.7 | 90.9 |

**Table 6: Impurity data of admixed carmustine solution with 0.9% sodium chloride injection (NaCl) and 5% dextrose injection**

| **Sampling intervals and storage condition** | **Related Substance (%)** | | | | | | | | | | | |
|---|---|---|---|---|---|---|---|---|---|---|---|---|
| | **PG** | | | | | | **DHA** | | | | | |
| | **NaCl** | | | **Dextrose** | | | **NaCl** | | | **Dextrose** | | |
| | **IA** | **IUUI** | **TI** | **IA** | **IUUI** | **TI** | **IA** | **IUUI** | **TI** | **IA** | **IUUI** | **TI** |
| **Stored at 24 Hrs. at 2°C-8°C and further for 12 hrs. at 25°C ± 2°C** | | | | | | | | | | | | |
| Initial (0) | 0.21 | 0.007 | 0.22 | 0.15 | 0.012 | 0.18 | 0.09 | 0.008 | 0.10 | 0.17 | 0.021 | 0.19 |
| 12hrs (2-8°C) | 0.25 | 0.009 | 0.27 | 0.22 | 0.022 | 0.28 | 0.16 | 0.010 | 0.17 | 0.18 | 0.031 | 0.22 |
| 24hrs (2-8°C) | 0.30 | 0.009 | 0.33 | 0.21 | 0.036 | 0.27 | 0.21 | 0.019 | 0.26 | 0.20 | 0.044 | 0.25 |
| 24hrs(2-8°C) + | 0.10 | 0.016 | 0.15 | 0.14 | 0.063 | 0.28 | 0.18 | 0.012 | 0.21 | 0.18 | 0.079 | 0.28 |
| 6hrs(25°C ±2°C) | | | | | | | | | | | | |
| 24hrs (2-8°C) + 12hrs(25°C±2°C) | 0.15 | 0.018 | 0.20 | 0.14 | 0.108 | 0.31 | 0.13 | 0.012 | 0.17 | 0.16 | 0.127 | 0.38 |

| **Stored at 48 Hrs. at 2°C-8°C and further for 12 hrs. at 25°C ± 2°C** | | | | | | | | | | | | |
|---|---|---|---|---|---|---|---|---|---|---|---|---|
| Initial (0hour) | 0.21 | ND | 0.21 | 0.16 | 0.025 | 0.21 | 0.15 | 0.009 | 0.16 | 0.22 | 0.030 | 0.25 |
| 48hrs (2-8°C) | 0.23 | 0.032 | 0.32 | 0.19 | 0.101 | 0.36 | 0.16 | 0.012 | 0.20 | 0.18 | 0.123 | 0.34 |
| 48hrs (2-8°C)+ 6hrs (25°C±2°C) | 0.14 | 0.027 | 0.20 | 0.16 | 0.142 | 0.39 | 0.18 | 0.015 | 0.22 | 0.17 | 0.174 | 0.40 |
| 48hrs (2-8°C)+12 hrs (25°C ±2°C) | 0.18 | 0.025 | 0.25 | 0.15 | 0.208 | 0.51 | 0.19 | 0.017 | 0.28 | 0.17 | 0.250 | 0.53 |

| **Stored at 8 hrs. at 25°C ±** 2°C | | | | | | | | | | | | |
|---|---|---|---|---|---|---|---|---|---|---|---|---|
| Initial (0 hour) | 0.27 | 0.028 | 0.33 | 0.15 | ND | 0.15 | 0.14 | 0.007 | 0.16 | ND | 0.007 | 0.01 |
| 4 hrs. (25°C ± 2°C) | 0.27 | 0.013 | 0.30 | 0.20 | 0.047 | 0.29 | 0.16 | 0.014 | 0.18 | 0.26 | 0.030 | 0.38 |
| 8 hrs. (25°C ± 2°C) | 0.25 | 0.037 | 0.34 | 0.23 | 0.085 | 0.37 | 0.21 | 0.012 | 0.24 | 0.25 | 0.092 | 0.38 |

As shown by the results in Tables 5 and 6, the admixed carmustine solution of Test Example 1 is stable (≥ 90% carmustine remaining) for 48 hours (2°C -8°C) + 6 hours (25°C ± 2°C).

## Claims

1. A method for preparing an administrable solution of carmustine for administration by intravenous infusion, said method comprising:
(a) dissolving 50-200 mg of lyophilized carmustine in 2-6 mL of sterile propylene glycol to form a reconstituted solution, and
(b) diluting the reconstituted solution with an aqueous 0.9% sodium chloride solution or an aqueous 5% dextrose solution to obtain the administrable solution.

2. The method of claim 1, wherein step (a) comprises dissolving 100 mg of lyophilized carmustine in 3 mL of sterile propylene glycol to form a reconstituted solution.

3. The method of claim 1 or 2, wherein prior to dissolving the lyophilized carmustine in the propylene glycol, (i) the lyophilized carmustine and propylene glycol are stored in separate vials at 2-8° C and either (ii) the propylene glycol is allowed to attain room temperature just prior to dissolving the lyophilized carmustine in the propylene glycol, or (ii') the vials are allowed to attain room temperature just prior to dissolving the lyophilized carmustine in the propylene glycol.

4. The method of claim 3, wherein the propylene glycol is aseptically removed from its vial with a sterile syringe having a needle below 22 gauge and injected into the vial containing the lyophilized carmustine.

5. The method of any one of claims 1-4, wherein the reconstituted solution contains at least 90% of the initial carmustine after storage at 2-8° C for up to 480 hours.

6. The method of any one of claims 1-5, wherein the reconstituted solution is stored at 2-8° C for up to 480 hours prior to step (b), and after storage at 2-8° C and prior to performing step (b), the reconstituted solution is examined for crystal formation and if crystals are observed, they are re-dissolved by warming the re-constituted solution to room temperature with agitation.

7. The method of any one of claims 1-6, wherein step (b) comprises diluting the reconstituted solution up to 500 mL with an aqueous 0.9% sodium chloride solution or 500 mL of an aqueous 5% dextrose solution to obtain the administrable solution.

8. The method of any one of claims 1-7, wherein step (b) is performed within 48 hours of the reconstituted solution being prepared, and/or wherein step (b) is performed more than 24 hours but less than 48 hours after the reconstituted solution is prepared.

9. The method of any one of claims 1-8, wherein the concentration of carmustine in the administrable solution is 0.2 mg/mL, and/or wherein administrable solution has a pH in the range of 6 to 7 and an osmolality in the range of 330-390 mOsmol/L.

10. A method for preparing an administrable solution of carmustine for administration by intravenous infusion, wherein the administrable solution is prepared from a kit comprising a product vial containing 100 mg of lyophilized carmustine and a diluent vial containing 3 mL of sterile propylene glycol, the kit being stored at 2-8° C, and wherein the administrable solution is prepared by:
(a) allowing the diluent vial to attain room temperature,
(b) aseptically removing the 3 mL of propylene glycol from the diluent vial, injecting it into the product vial containing 100 mg of lyophilized carmustine, and shaking the product vial to dissolve the lyophilized carmustine to form a reconstituted solution, wherein, preferably, the lyophilized carmustine from the product vial is reconstituted with specified amount of propylene glycol from the diluent vial in a single-step reconstitution procedure,
(c) optionally, storing the reconstituted solution and prior to performing step (d), the stored reconstituted solution is examined for crystal formation and if crystals are observed, they are re-dissolved by warming the reconstituted solution to room temperature with agitation, and
(d) diluting the reconstituted solution up to 500 mL with an aqueous 0.9% sodium chloride solution or an aqueous 5% dextrose solution to obtain the administrable solution.

11. The method of claim 10, wherein the propylene glycol is removed from the diluent vial in step (b) with a below 22-gauge needle.

12. A kit for preparing an injectable carmustine solution, the kit comprising a product vial containing lyophilized carmustine and a diluent vial containing only sterile propylene glycol, wherein, upon dissolving the lyophilized carmustine in the propylene glycol, the resulting solution contains at least 90% of the initial carmustine after storage at 2-8° C for up to 720 hours or at 25 °C ± 2 °C for up to 48 hours.

13. The kit according to claim 12, wherein the concentration of lyophilized carmustine in product vial is from about 2 mg/vial to about 500 mg/vial, wherein, preferably, the product vial contains 50-200 mg of lyophilized carmustine and the diluent vial contains 2-4 mL of sterile propylene glycol.

14. The kit according to claim 12 or 13, wherein the amount of sterile propylene glycol in the diluent vial is between 1 ml and 20 ml, preferably 2-4 mL.

## Patentansprüche

1. Verfahren zum Herstellen einer verabreichbaren Lösung von Carmustin für eine Verabreichung durch intravenöse Infusion, wobei das Verfahren umfasst:
(a) Lösen von 50-200 mg lyophilisiertem Carmustin in 2-6 mL sterilem Propylenglykol, um eine rekonstituierte Lösung zu bilden, und
(b) Verdünnen der rekonstituierten Lösung mit einer wässrigen 0,9 % Natriumchloridlösung oder einer wässrigen 5 % Dextrose-Lösung, um die verabreichbare Lösung zu erhalten.

2. Verfahren nach Anspruch 1, wobei Schritt (a) ein Lösen von 100 mg lyophilisiertem Carmustin in 3 mL sterilem Propylenglykol umfasst, um eine rekonstituierte Lösung zu bilden.

3. Verfahren nach Anspruch 1 oder 2, wobei, vor dem Lösen des lyophilisierten Carmustins in dem Propylenglykol, (i) das lyophilisierte Carmustin und Propylenglykol in separaten Fläschchen bei 2-8° C gelagert werden und entweder (ii) es dem Propylenglykol kurz vor dem Lösen des lyophilisierten Carmustins in dem Propylenglykol ermöglicht wird, Raumtemperatur anzunehmen, oder (ii') es den Fläschchen kurz vor dem Lösen des lyophilisierten Carmustins in dem Propylenglykol ermöglicht wird, Raumtemperatur anzunehmen.

4. Verfahren nach Anspruch 3, wobei das Propylenglykol mit einer sterilen Spritze mit einer Nadel unter 22 Gauge aseptisch aus seinem Fläschchen entnommen und in das Fläschchen, das das lyophilisierte Carmustin enthält, injiziert wird.

5. Verfahren nach einem der Ansprüche 1-4, wobei die rekonstituierte Lösung nach einer Lagerung bei 2-8° C für bis zu 480 Stunden mindestens 90% des ursprünglichen Carmustins enthält.

6. Verfahren nach einem der Ansprüche 1-5, wobei die rekonstituierte Lösung vor Schritt (b) bei 2-8° C für bis zu 480 Stunden gelagert wird, und nach einer Lagerung bei 2-8° C und vor dem Durchführen von Schritt (b) die rekonstituierte Lösung auf Kristallbildung untersucht wird und, falls Kristalle beobachtet werden, diese durch Erwärmen der rekonstituierten Lösung auf Raumtemperatur unter Rühren wieder gelöst werden.

7. Verfahren nach einem der Ansprüche 1-6, wobei Schritt (b) ein Verdünnen der rekonstituierten Lösung auf bis zu 500 mL mit einer wässrigen 0,9% Natriumchloridlösung oder 500 mL einer wässrigen 5% Dextrose-Lösung umfasst, um die verabreichbare Lösung zu erhalten.

8. Verfahren nach einem der Ansprüche 1-7, wobei Schritt (b) innerhalb von 48 Stunden nach einem Herstellen der rekonstituierten Lösung durchgeführt wird, und/oder wobei Schritt (b) mehr als 24 Stunden, aber weniger als 48 Stunden nach Herstellung der rekonstituierten Lösung durchgeführt wird.

9. Verfahren nach einem der Ansprüche 1-8, wobei die Konzentration von Carmustin in der verabreichbaren Lösung 0,2 mg/mL ist, und/oder wobei die verabreichbare Lösung einen pH in dem Bereich von 6 bis 7 und eine Osmolalität in dem Bereich von 330-390 mOsmol/L hat.

10. Verfahren zum Herstellen einer verabreichbaren Lösung von Carmustin für eine Verabreichung durch intravenöse Infusion, wobei die verabreichbare Lösung aus einem Kit hergestellt wird, umfassend ein Produktfläschchen, das 100 mg lyophilisiertes Carmustin enthält, und ein Verdünnungsmittelfläschchen, das 3 mL steriles Propylenglykol enthält, wobei das Kit bei 2-8° C gelagert wird, und wobei die verabreichbare Lösung hergestellt wird durch:
(a) Ermöglichen, dass das Verdünnungsmittelfläschchen Raumtemperatur annimmt,
(b) aseptisches Entnehmen der 3 mL Propylenglykol aus dem Verdünnungsmittelfläschchen, Injizieren derselben in das Produktfläschchen, das 100 mg lyophilisiertes Carmustin enthält, und Schütteln des Produktfläschchens, um das lyophilisierte Carmustin zu lösen, um eine rekonstituierte Lösung zu bilden, wobei, bevorzugt, das lyophilisierte Carmustin aus dem Produktfläschchen mit einer bestimmten Menge Propylenglykol aus dem Verdünnungsmittelfläschchen in einem einstufigen Rekonstitutionsverfahren rekonstituiert wird,
(c) wahlweise, Lagern der rekonstituierten Lösung und, vor einem Durchführen von Schritt (d), Untersuchen der gelagerten rekonstituierten Lösung auf Kristallbildung, und, falls Kristalle beobachtet werden, werden diese durch Erwärmen der rekonstituierten Lösung auf Raumtemperatur unter Rühren wieder gelöst, und
(d) Verdünnen der rekonstituierten Lösung auf bis zu 500 mL mit einer wässrigen 0,9% Natriumchloridlösung oder einer wässrigen 5% Dextrose-Lösung, um die verabreichbare Lösung zu erhalten.

11. Verfahren nach Anspruch 10, wobei das Propylenglykol aus dem Verdünnungsmittelfläschchen in Schritt (b) mit einer Nadel unter 22 Gauge entnommen wird.

12. Kit zum Herstellen einer injizierbaren Carmustinlösung, wobei das Kit ein Produktfläschchen, das lyophilisiertes Carmustin enthält, und ein Verdünnungsmittelfläschchen, das nur steriles Propylenglykol enthält, umfasst, wobei die bei einem Lösen des lyophilisierten Carmustins in dem Propylenglykol entstehende Lösung nach einer Lagerung bei 2-8°C für bis zu 720 Stunden oder bei 25°C ± 2°C für bis zu 48 Stunden mindestens 90% des ursprünglichen Carmustins enthält.

13. Kit nach Anspruch 12, wobei die Konzentration von lyophilisiertem Carmustin in dem Produktfläschchen von etwa 2 mg/Fläschchen bis etwa 500 mg/Fläschchen ist, wobei, bevorzugt, das Produktfläschchen 50-200 mg lyophilisiertes Carmustin enthält und das Verdünnungsmittelfläschchen 2-4 mL steriles Propylenglykol enthält.

14. Kit nach Anspruch 12 oder 13, wobei die Menge von sterilem Propylenglykol in dem Verdünnungsmittelfläschchen zwischen 1 mL und 20 mL, bevorzugt 2-4 mL, ist.

## Revendications

1. Procédé pour préparer une solution administrable de carmustine pour une administration par perfusion intraveineuse, ledit procédé comprenant :
(a) la dissolution de 50 à 200 mg de carmustine lyophilisée dans 2 à 6 ml de propylèneglycol stérile pour former une solution reconstituée, et
(b) la dilution de la solution reconstituée avec une solution aqueuse à 0,9 % de chlorure de sodium ou une solution aqueuse à 5 % de dextrose pour que soit obtenue la solution administrable.

2. Procédé selon la revendication 1, dans lequel l'étape (a) comprend la dissolution de 100 mg de carmustine lyophilisée dans 3 ml de propylèneglycol stérile pour former une solution reconstituée.

3. Procédé selon la revendication 1 ou 2, dans lequel, avant la dissolution de la carmustine lyophilisée dans le propylèneglycol, (i) la carmustine lyophilisée et le propylèneglycol sont stockés dans des flacons séparés à 2-8°C et soit (ii) le propylèneglycol est laissé à atteindre la température ambiante juste avant la dissolution de la carmustine lyophilisée dans le propylèneglycol, soit (ii') les flacons sont laissés à atteindre la température ambiante juste avant la dissolution de la carmustine lyophilisée dans le propylèneglycol.

4. Procédé selon la revendication 3, dans lequel le propylèneglycol est soutiré de manière aseptique hors de son flacon avec une seringue stérile ayant une aiguille de taille inférieure au calibre 22 et injecté dans le flacon contenant la carmustine lyophilisée.

5. Procédé selon l'une quelconque des revendications 1 à 4, dans lequel la solution reconstituée contient au moins 90 % de la carmustine initiale après jusqu'à 480 heures de stockage à 2-8°C.

6. Procédé selon l'une quelconque des revendications 1 à 5, dans lequel la solution reconstituée est stockée à 2-8°C pendant jusqu'à 480 heures avant l'étape (b) et, après stockage à 2-8°C et avant réalisation de l'étape (b), la formation de cristaux est examinée dans la solution reconstituée, et si des cristaux sont observés, ils sont re-dissous par réchauffage de la solution reconstituée à la température ambiante sous agitation.

7. Procédé selon l'une quelconque des revendications 1 à 6, dans lequel l'étape (b) comprend la dilution de la solution reconstituée à 500 ml avec une solution aqueuse à 0,9 % de chlorure de sodium ou 500 ml d'une solution aqueuse à 5 % de dextrose pour que soit obtenue la solution administrable.

8. Procédé selon l'une quelconque des revendications 1 à 7, dans lequel l'étape (b) est réalisée dans les 48 heures suivant la préparation de la solution reconstituée, et/ou dans lequel l'étape (b) est réalisée plus de 24 heures mais moins de 48 heures après la préparation de la solution reconstituée.

9. Procédé selon l'une quelconque des revendications 1 à 8, dans lequel la concentration de carmustine dans la solution administrable est de 0,2 mg/ml, et/ou dans lequel la solution administrable a un pH situé dans la plage allant de 6 à 7 et une osmolalité située dans la plage allant de 330 à 390 mOsmol/l.

10. Procédé pour préparer une solution administrable de carmustine pour une administration par perfusion intraveineuse, dans lequel la solution administrable est préparée à partir d'une trousse comprenant un flacon de produit contenant 100 mg de carmustine lyophilisée et un flacon de diluant contenant 3 ml de propylèneglycol stérile, la trousse étant stockée à 2-8°C, et dans lequel la solution administrable est préparée par :
(a) le fait de laisser le flacon de diluant atteindre la température ambiante,
(b) le soutirage aseptique des 3 ml de propylèneglycol hors du flacon de diluant, l'injection de ceux-ci dans le flacon de produit contenant 100 mg de carmustine lyophilisée, et le secouage du flacon de produit pour dissoudre la carmustine lyophilisée pour former une solution reconstituée, dans lequel de préférence la carmustine lyophilisée du flacon de produit est reconstituée avec une quantité spécifiée de propylèneglycol provenant du flacon de diluant dans une procédure de reconstitution en une seule étape,
(c) éventuellement le stockage de la solution reconstituée et, avant la réalisation de l'étape (d), la formation de cristaux est examinée dans la solution reconstituée, et si des cristaux sont observés, ils sont re-dissous par réchauffage de la solution reconstituée à la température ambiante sous agitation, et
(d) la dilution de la solution reconstituée à 500 ml avec une solution aqueuse à 0,9 % de chlorure de sodium ou une solution aqueuse à 5 % de dextrose pour que soit obtenue la solution administrable.

11. Procédé selon la revendication 10, dans lequel le propylèneglycol est soutiré du flacon de diluant dans l'étape (b) avec une aiguille de taille inférieure au calibre 22.

12. Trousse pour préparer une solution de carmustine injectable, la trousse comprenant un flacon de produit contenant de la carmustine lyophilisée et un flacon de diluant contenant uniquement du propylèneglycol stérile, dans laquelle, suite à la dissolution de la carmustine lyophilisée dans le propylèneglycol, la solution résultante contient au moins 90 % de la carmustine initiale après jusqu'à 720 heures de stockage à 2-8°C ou jusqu'à 48 heures de stockage à 25°C ± 2°C.

13. Trousse selon la revendication 12, dans laquelle la concentration de carmustine lyophilisée dans le flacon de produit est d'environ 2 mg/flacon à environ 500 mg/flacon, dans laquelle, de préférence, le flacon de produit contient 50 à 200 mg de carmustine lyophilisée et le flacon de diluant contient 2 à 4 ml de propylèneglycol stérile.

14. Trousse selon la revendication 12 ou 13, dans laquelle la quantité de propylèneglycol stérile dans le flacon de diluant est comprise entre 1 ml et 20 ml, de préférence de 2 à 4 ml.
